# EUROPÄISCHE PATENTSCHRIFT

(11) **EP 1 871 428 B1**
(45) Veröffentlichungstag und Bekanntmachung des Hinweises auf die Patenterteilung: **11.03.2009**
(21) Anmeldenummer: 06724367.5
(22) Anmeldetag: 15.04.2006
(51) Int. Cl.: A61L 2/00, B01J 19/08

(54) **OBERFLÄCHENBEHANDLUNGSANLAGE**
SURFACE TREATMENT SYSTEM
INSTALLATION DE TRAITEMENT DE SURFACE

(30) Priorität: 22.04.2005 DE 102005019700
(43) Veröffentlichungstag der Anmeldung: 02.01.2008
(73) Patentinhaber: EISENMANN Anlagenbau GmbH & Co. KG, 71032 Böblingen (DE)
(72) Erfinder: LINK, Kersten, 71120 Grafenau (DE); SWOBODA, Werner, 71032 Böblingen (DE)
(74) Vertreter: Schwanhäusser, Gernot
(86) Internationale Anmeldenummer: PCT/EP2006/003496
(87) Internationale Veröffentlichungsnummer: WO 2006/111337

(56) Entgegenhaltungen:
- US-A- 5 611 993
- US-A1- 2002 144 957
- US-A1- 2004 226 823

## Beschreibung

Die Erfindung betrifft eine Oberflächenbehandlungsanlage, insbesondere zum Lackieren, Beschichten, Trocknen und damit verbundenen Vorbereiten von metallischen oder nichtmetallischen Gegenständen, mit einem Kreislauf, in dem eine Flüssigkeit umgewälzt wird.

Derartige Oberflächenbehandlungsanlagen sind allgemein im Stand der Technik bekannt siehe auch US 2004/00226823. Sie dienen dazu, Oberflächen von Gegenständen auf unterschiedliche Weise, z.B. durch Aufbringen von Lacken und anderen Beschichtungen, zu behandeln. Im allgemeinen enthalten derartige Anlagen mehrere einzelne Behandlungsstationen für unterschiedliche Behandlungsschritte, z.B. Vorbereiten, Lackieren und Trocknen. Die zu behandelnden Gegenstände, bei denen es sich um metallische, aber auch um nicht-metallische Gegenstände handeln kann, werden zu diesem Zweck mit Hilfe eines Fördersystems von Behandlungsstation zu Behandlungsstation gefördert.

In den einzelnen Behandlungsstationen werden häufig größere Flüssigkeitsmengen umgewälzt. Die Flüssigkeiten werden z.B. zur Reinigung der Stationen, zum Entfetten oder Spülen der Gegenstände oder als Träger für Pigmente verwendet. Aus Kosten- und Umweltgründen werden diese Flüssigkeiten im allgemeinen nach ihrer erstmaligen Verwendung nicht entsorgt, sondern in einem Kreislauf umgewälzt und dabei einer Wiederaufbereitungseinrichtung zugeführt. In der Wiederaufbereitungseinrichtung findet im allgemeinen eine mechanische und chemische-physikalische Reinigung statt, bevor die Flüssigkeit wieder verwendet wird. Auf diese Weise muß eine einmal bereitgestellte Flüssigkeit nicht mehr vollständig ausgetauscht werden. Ein Ersatz findet im allgemeinen lediglich in der Weise statt, daß kontinuierlich oder in regelmäßigen Abständen kleinere Flüssigkeitsmengen zugeführt werden, die Flüssigkeitsverluste, z.B. infolge eines Austrags der Flüssigkeit durch die Gegenstände oder durch Verdunstung, ausgleichen.

Aufgrund der langen Verweildauer der Flüssigkeiten in den Kreisläufen können sich in der Flüssigkeit Keime vermehren. Besonders schnell vermehren sich die Keime, wenn die Flüssigkeit warm ist, wie dies häufig z.B. beim kataphoretischen Tauchlackieren der Fall ist. Unter Keimen werden im vorliegenden Zusammenhang neben Bakterien und anderen Einzellern auch Pilze und Algen verstanden.

Bei ungehemmter Vermehrung können derartige Keime zu ernsten gesundheitlichen Beeinträchtigungen des Bedienpersonals führen und sogar eine Abschaltung der Anlage erforderlich machen. Besonders leicht können Keime beim Versprühen von Flüssigkeiten in die Luft übergehen, wie dies beispielsweise bei der Reinigung von Spritzkabinen der Fall ist.

Ferner besteht die Gefahr, daß sich die Keime an Oberflächen sammeln und so z.B. Filter oder Rohre mit kleinen Durchmessern zusetzen. Falls sich die Keime auf den Oberflächen der zu behandelnden Gegenstände absetzen, können auch Beeinträchtigungen des technischen Ergebnisses, z.B. Lackschäden, auftreten. Da die Keime bei der Förderung der Gegenständen von Station zu Station übertragen werden, besteht auch die Gefahr, daß Keime in Bereiche eingetragen werden, in denen eine Vermehrung an sich wegen ungünstiger chemischer oder thermischer Verhältnisse eher unwahrscheinlich ist. Wird beispielsweise der Inhalt eines Lacktauchbeckens durch Keime verunreinigt, so kann dies einen sehr teuren Austausch der dort enthaltenen Flüssigkeit erforderlich machen.

Da sich relativ schnell hohe Keimkonzentrationen von mehr als 10⁸ Keimen pro cm³ einstellen können, mischt man den Flüssigkeiten zur Sterilisation Biozide ein, worunter man Bakterizide und Fungizide versteht. Durch die bioaktiv toxischen Substanzen lassen sich zwar die Keimkonzentrationen relativ gering halten, jedoch sind die Kosten für diese Art der Entkeimung hoch. Außerdem stellen die Biozide Zusatzstoffe dar, die ebenfalls das technische Ergebnis der Behandlung beeinträchtigen können und die biologische Klärung von Abwässern erschweren. Ein weiteres Problem beim Einsatz von chemisch-biologischen Mitteln ist die Fähigkeit zahlreicher Keime, resistente Stämme zu entwickeln, die - wenn überhaupt - nur mit neuen und deswegen besonders teuren Mitteln wirksam bekämpft werden können.

Vor diesem Hintergrund ist es Aufgabe der vorliegenden Erfindung, eine Oberflächenbehandlungsanlage derart zu verbessern, daß in umgewälzten Flüssigkeiten einfach und kostengünstig eine Verringerung der Keimkonzentration erzielt werden kann.

Erfindungsgemäß wird diese Aufgabe dadurch gelöst, daß zum Zwecke der Entkeimung der Flüssigkeit eine Einrichtung zum mechanischen Öffnen von Zellmembranen in dem Kreislauf integriert ist.

Die Keime werden somit nicht chemisch-biologisch abgetötet, sondern mechanisch derart beansprucht, daß sich die Zellmembranen irreversibel öffnen. Dadurch tritt das Zytoplasma aus den Zellen aus, was zu deren Absterben führt. Diese Art von Entkeimung hat den Vorteil, daß - abgesehen von dem getöteten biologischen Material - keine Rückstände in der Flüssigkeit verbleiben, wie dies bei der Behandlung mit Bioziden der Fall ist. Ferner läßt sich eine solche mechanische Zerstörung der Keime vergleichsweise kostengünstig und effizient durchführen. Ein weiterer Vorteil dieses Ansatzes besteht darin, daß sich die Keime nicht durch Erzeugung resistenter Stämme der letztlich mechanischen Sterilisation entziehen können, wie dies bei der chemisch-biologischen Sterilisation mit Hilfe von Bioziden der Fall ist. Schließlich funktioniert die mechanische Öffnung von Zellmembranen zum Zwecke der Entkeimung auch dann, wenn die Flüssigkeit trübe ist oder stark absorbierende Pigmente enthält. Dies ist ein bedeutender Vorteil gegenüber der Bestrahlung mit kurzwelliger elektromagnetischer Strahlung, z.B. UV-Licht, die bislang ebenfalls zur Entkeimung eingesetzt wird.

Bei der Einrichtung zum mechanischen Öffnen von Zellmembranen kann es sich beispielsweise um eine Elektroporationseinrichtung handeln. Der Begriff "Elektroporation" bezeichnet ein Verfahren, bei dem die Zellen kurzzeitig starken elektrischen Feldern ausgesetzt werden. Feinste Poren, die in der Zellmembran bereits vorhanden sind, werden unter dem Einfluß des elektrischen Felds derart aufgeweitet, daß sie sich auch nach Abklingen des elektrischen Feldes nicht wieder schließen. Voraussetzung hierfür ist lediglich, daß das elektrische Feld eine ausreichende Feldstärke hat und über eine gewisse Mindestdauer hinweg besteht.

Diese Art der Abtötung biologischer Zellen ist an und für sich bekannt aus einem Aufsatz von H. Bluhm et al. mit dem Titel "Aufschluß und Abtötung biologischer Zellen mit Hilfe starker gepulster elektrische Felder", Nachrichten - Forschungszentrum Karlsruhe, Jahrgang 35, 3/2003, Seite 105 bis 110. Im Vordergrund bei der Sterilisation steht bislang die Reinigung von Abwässern aus Kläranlagen, wie dies beispielsweise in der US 2002/0144957 A1 beschrieben ist. Allerdings ist die Abtötung von Bakterien und anderen Mikroorganismen durch Elektroporation schwieriger als die Öffnung pflanzlicher Zellen, wie sie beispielsweise in industriellen Entsaftern eingesetzt wird.

Die Erfinder haben entdeckt, daß die Schwierigkeiten, wie sie bei der Elektroporation biologischer Abwässer beschrieben wurden, in Oberflächenbehandlungsanlagen nicht oder nur in begrenztem Umfang auftreten. Dies hängt beispielsweise damit zusammen, daß in die Oberflächenstationen von außen nur relativ geringe Mengen an biologischem Material eingetragen werden. Vor allem jedoch werden die Flüssigkeiten relativ häufig umgewälzt, so daß selbst vergleichsweise kleine Entkeimungsraten ausreichen, um die Keimkonzentration auf einem sehr niedrigen Wert zu halten.

Anstelle einer Elektroporationseinrichtung kann auch eine Kavitationseinrichtung verwendet werden, welche die Flüssigkeit derart beschleunigt, daß durch Kavitation hervorgerufene Druckstöße die Zellmembranen öffnen. Unter dem Begriff der Kavitation versteht man die Bildung von gasgefüllten Hohlräumen in Flüssigkeiten in Unterdruckbereichen, wie sie beispielsweise entstehen, wenn der momentane örtliche Druck den Dampfdruck der Flüssigkeit unterschreitet. Sinkt bei Beschleunigung einer strömenden Flüssigkeit der Druck unter den Dampfdruck ab, so bilden sich Dampfbläschen, die bei Druckanstieg durch Implosion kondensieren. In Folge der damit einhergehenden plötzlichen Volumenänderung können Druckstöße bis 10 000 bar auftreten, welche die Zellmembranen zerstören.

Zur Erzeugung der Kavitation muß der statische Druck der Flüssigkeit abgesenkt werden. Dies kann durch Beschleunigen der Flüssigkeit erreicht werden, wie sie beispielsweise beim Durchtritt der Flüssigkeit durch eine Engstelle eintritt. Eine Beschleunigung kann auch durch Kontakt mit sich schnell bewegenden Teilen, z.B. einem Rotor einer Pumpe, erzielt werden.

Bei dem Kreislauf kann es sich insbesondere um einen Teil einer Wiederaufbereitungseinrichtung zum Wiederaufbereiten der Flüssigkeit handeln. Die Wiederaufbereitungseinrichtung kann wiederum einer oder mehrerer Bearbeitungsstationen zugeordnet sein. In Vorbehandlungsstationen, z.B. einer Entfettungsstation oder einer Sprüh- oder Tauch-Spülstation, wird häufig mit warmen Spülflüssigkeiten gearbeitet, in denen Keime gute Voraussetzungen für eine Vermehrung finden. Auch in der Vorbehandlung nachgeordneten Tauch- oder Spritzlackierstationen sind in aller Regel Wiederaufbereitungseinrichtungen zur Regenerierung umgewälzter Flüssigkeiten, und zwar sowohl von Lacken als auch von Lackauswaschwässern, vorgesehen. In einem Bad zur kataphoretischen Tauchlackierung herrscht z.B. ebenfalls eine höhere Temperatur, und eine Verkeimung des Badinhalts ist besonders kritisch, da ein Austausch von Lacken erhebliche Kosten verursacht.

Weitere Merkmale und Vorteile der Erfindung ergeben sich aus der nachfolgenden Beschreibung eines Ausführungsbeispiels anhand der Zeichnung. Darin zeigen:
- Figur 1: ein Blockschaltbild eines Vorbehandlungsbe- reichs einer Lackierstraße;
- Figur 2: eine Tauch-Entfettungs-Station des in der Figur 1 gezeigten Vorbehandlungsbereichs in einem schematischen Längsschnitt;
- Figur 3: eine Sprühspül-Station des in der Figur 1 ge- zeigten Vorbehandlungsbereichs in einem schema- tischen Längsschnitt;
- Figur 4: einen vereinfachten Querschnitt durch eine Elektroporationseinrichtung, in der eine Koro- narentladung erzeugt wird.

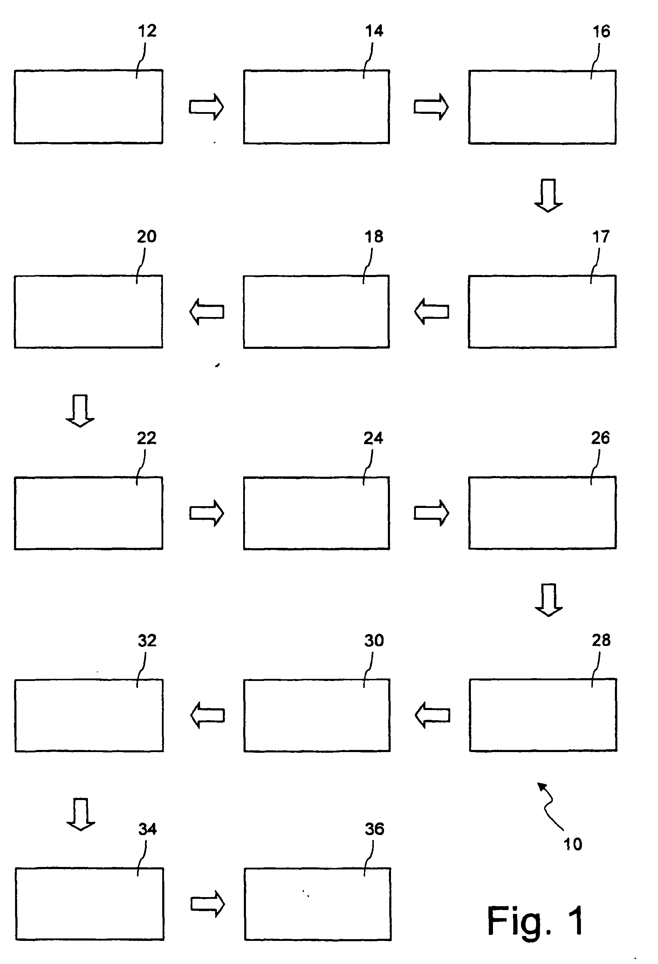

Die Figur 1 zeigt in Form eines Blockschaltbildes einen insgesamt mit 10 bezeichneten Vorbehandlungsbereich einer Lackierstraße, in der Rohkarosserien für Kraftfahrzeuge lackiert werden. Dabei sei angenommen, daß die vormontierten Rohkarosserien mit Hilfe eines nicht dargestellten Fördersystems in der durch Pfeile angegebenen Reihenfolge von Station zu Station gefördert und dort in unterschiedlicher Weise behandelt werden.

Da die Blechteile, aus denen die Rohkarosserien gefertigt werden, vor dem Preßziehen eingefettet werden, sind die Rohkarosserien mit einem dünnen Fettfilm überzogen, wenn sie in den Vorbehandlungsbereich 10 gelangen. Zum Entfetten der Rohkarosserien sind drei Stationen 12, 14 und 16 vorgesehen, in denen eine Schwall-Entfettung, eine Spritz-Entfettung bzw. eine Tauch-Entfettung in an sich bekannter Weise durchgeführt wird.

An die Entfettungs-Stationen 12, 14, 16 schließen sich zwei Spülstationen 17, 18 an. In den Stationen 20 und 22 findet eine Tauch-Aktivierung bzw. eine Zink-Phosphatierung statt. Daran schließen sich drei Stationen 24, 26, 28 an, in denen die Rohkarosserien mit entsalztem Wasser gespült werden. In der Lackier-Station 30 findet eine kataphoretische Tauchlackierung statt. Dort wird die Rohkarosserie in ein Lackbad eingetaucht und in einem elektrischen Feld beschichtet. An die Lackier-Station 30 schließen sich zwei Ultrafiltrations-Spülstationen 32, 34 sowie eine Sprühspül-Station 36 an, in der die Rohkarosserie erneut mit entsalzten Wasser gereinigt wird. Die Vorbehandlung der Rohkarosserien im Vorbehandlungsbereich 10 der Lackierstraße ist damit beendet.

Die Rohkarosserien werden dann getrocknet, spritzlakkiert, erneut getrocknet und ggf. weiteren Behandlungen unterzogen, bevor sie die Lackierstraße verlassen.

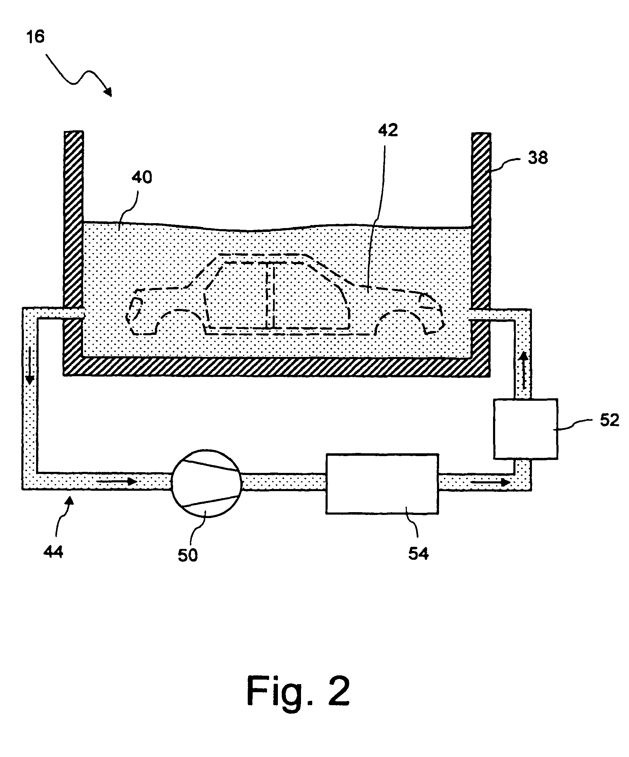

In der Figur 2 ist die Tauch-Entfettungs-Station 16 in einem schematischen Längsschnitt gezeigt. Die Tauch-Entfettungs-Station 16 umfaßt ein Tauchbecken 38, das mit erwärmten Wasser 40 gefüllt ist. Dem Wasser 40 sind Detergenzien beigefügt, die das Ablösen der Fettspuren von einer eingetauchten Rohkarosserie 42 unterstützen. Über einen Kreislauf 44 wird das Wasser 40 kontinuierlich mit Hilfe einer Pumpe 50 umgewälzt, wie dies durch Pfeile angedeutet ist. Das Wasser 40 durchläuft dabei eine Heizeinrichtung 52, die das Wasser 40 erwärmt. In dem Kreislauf 44 können noch weitere Aggregate integriert sein. In Betracht kommen hierzu beispielsweise Filterelemente oder Zuführeinrichtungen, mit denen sich ein Detergenz oder Wasser hinzufügen läßt, das durch die Rohkarosserien 42 aus dem Tauchbecken 38 ausgetragenes Wasser ersetzt.

In dem erwärmten und Fettspuren enthaltenden Wasser 40 finden Bakterien und andere Keime günstige Voraussetzungen für eine rasche Vermehrung. Durch die Förderung der Karosserien 42 können solche Keime in die nachfolgenden Behandlungsstationen eingetragen werden, in denen sich die Keime u.U. weiter vermehren.

Überschreitet die Keimkonzentration eine gewissen Größenordnung, so können die Keime kleine Öffnungen in Filtern o.ä. oder Rohre mit geringem Querschnitt zusetzen und dadurch Funktionsstörungen hervorrufen. Ferner besteht die Gefahr, daß sich die Keime auf der Rohkarosserie 42 ablagern und das Behandlungsergebnis beeinträchtigen.

Nach der Behandlung in dem Tauchbecken 38 wird die Rohkarosserie 42 aus dem Wasser 40 herausgehoben. Dabei kommt die Rohkarosserie 42 mit der umgebenden Luft in Berührung. Keime, die sich auf der Rohkarosserie 42 befinden, können auf diese Weise in die Luft übergehen und zu gesundheitlichen Beeinträchtigungen des Bedienpersonals führen. Besonders gefährliche Erreger, wie beispielsweise Legionellen, können sogar eine Stillegung der gesamten Lackierstraße erforderlich machen.

Um diese Gefahren und Beeinträchtigungen zu verringen, ist zum Zwecke der Entkeimung des Wassers 40 eine Elektroporationseinrichtung 54 in den Kreislauf 44 integriert. Bei dem in der Figur 2 dargestellten Ausführungsbeispiel befindet sich die Elektroporationseinrichtung 54 zwischen der Pumpe 50 und der Heizeinrichtung 52. Die Elektroporationseinrichtung 54 kann jedoch auch an anderer Stelle, z.B. in Fließrichtung vor der Pumpe 50, hinter der Heizeinrichtung 52 oder in einer (ggf. eigens hierfür vorgesehenen) Bypass-Leitung angeordnet sein.

Hierfür geeignete Elektroporationseinrichtungen sind an und für sich aus dem Stand der Technik bekannt. In diesem Zusammenhang wird auf den eingangs erwähnten Aufsatz von H. Bluhm et al. sowie auf die DE 101 44 486 C1 verwiesen. Die bei der Elektroporation wählbaren Parameter wie Impulsamplitude, Impulsdauer, Impulsfrequenz sowie Impulsform beeinflussen die Effizienz der Keimabtötung und sind an die jeweiligen Verhältnisse anzupassen. Da das Wasser 40 kontinuierlich in dem Kreislauf 44 umgewälzt wird, besteht die Möglichkeit, einen oder mehrere dieser Parameter während der Betriebsdauer der Lackierstraße zu verändern. Dadurch können auch ganz unterschiedliche Keime abgetötet werden.

Infolge der Elektroporation kann die Keimdichte des von der Pumpe 50 zugeführten Wassers 40 um mehrere Größenordnungen verringert werden. Infolge des kontinuierlichen Umwälzens läßt sich somit die Keimdichte auf einem so niedrigen Wert halten, daß weder Beeinträchtigungen des technischen Ergebnisses noch Gesundheitsgefährdungen zu erwarten sind.

Anstelle der Elektroporationseinrichtung 54 kann auch eine Kavitationseinrichtung vorgesehen sein, in der das Wasser 40 z.B. im Bereich einer Leitungsengstelle oder mit Hilfe eines Flügelrads o.ä. stark beschleunigt wird. Infolge der starken Beschleunigung entstehen Gasblasen in dem Wasser 40, die wiederum beim Kondensieren starke Druckstöße erzeugen. Diese Druckstöße öffnen zumindest teilweise die Zellmembranen der Keime, wodurch ein ähnlicher Effekt wie in der Elektroporationseinrichtung erzielt wird.

Da in allen in der Figur 1 gezeigten Stationen Flüssigkeiten verwendet, aus dem eigentlichen Behandlungsbereich ausgeleitet und in einem Kreislauf wieder aufbereitet werden, können auch dort Elektroporations- oder Kavitationseinrichtungen verwendet werden, wie dies vorstehend mit Bezug auf die Figur 2 erläutert wurde.

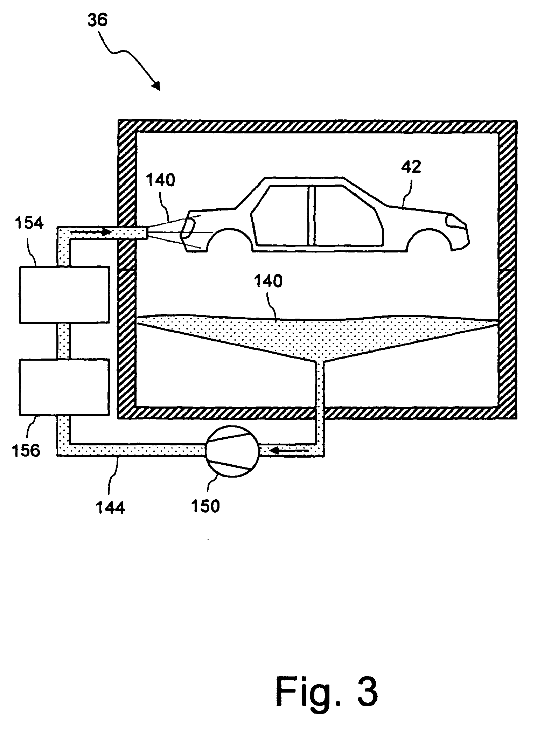

Die Figur 3 zeigt stellvertretend für diese weiteren Stationen die Sprühspül-Station 36 in einem stark schematisierten Längsschnitt, in der die Fahrzeugkarosserien nach dem Ultrafiltrations-Spülen in den Stationen 32, 34 mit entsalztem Wasser 140 abgesprüht werden. Das Wasser 140 sammelt sich am Boden der Station 36 und wird in einem Kreislauf 144 wieder aufbereitet. Das Wasser 144 durchläuft dabei eine Pumpe 150, eine Filter- und Entsalzungseinrichtung 156 sowie eine Elektroporationseinrichtung 154, bevor es wieder auf die Rohkarosserien 42 aufgesprüht wird.

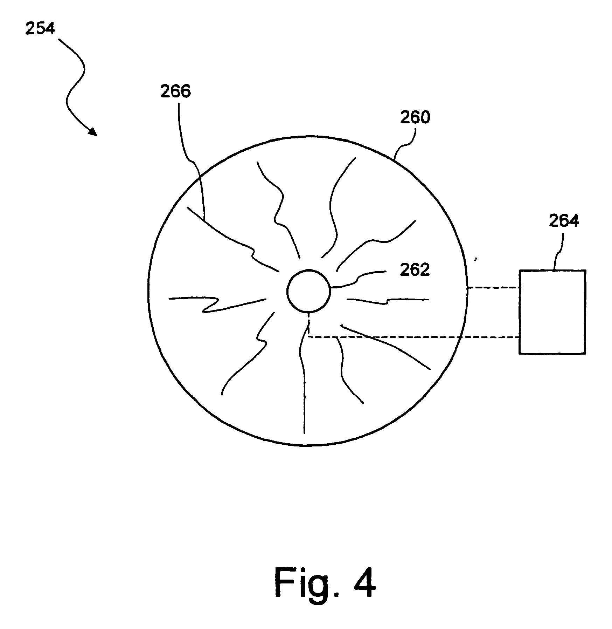

Die Figur 4 zeigt einen Querschnitt durch wesentliche Teile einer Elektroporationseinrichtung, mit der sich zusätzlich Koronarentladungen erzeugen lassen. In einem ersten Rohr 260 mit Durchmesser d₁ ist koaxial ein zweites Rohr 262 mit Durchmesser d₂ < d₁ angeordnet. Die beiden Rohre 260, 262 bilden die Elektroden der Elektroporationseinrichtung 254. Die Rohre 260, 262 sind mit einem Impulsgenerator 264 verbunden, mit dem sich Hochspannungsimpulse erzeugen lassen.

Fließt eine zu entkeimende Flüssigkeit durch den Zwischenraum zwischen den beiden Rohren 260, 262, so kommt es bei Erzeugung von ausreichend hohen Feldstärken zwischen den Rohren 260, 262 zur Ausbildung von Koronarentladungen, die in der Figur 4 mit Linien 266 angedeutet sind. Die Koronarentladungen 266 verstärken die Entkeimung der durch den Zwischenraum fließenden Flüssigkeit. Infolge der Koronarentladungen 266 entstehen nämlich in der Flüssigkeit freie Radikale und andere chemisch aggressive Stoffe wie etwa H₂O₂, welche auf chemischbiologischem Wege die Keime angreifen.

## Patentansprüche

1. Oberflächenbehandlungsanlage, insbesondere zum Lackieren, Beschichten, Trocknen und damit verbundenen Vorbereiten von metallischen oder nichtmetallischen Gegenständen (42), mit einem Kreislauf (44; 144), in dem eine Flüssigkeit (40; 140) umgewälzt wird,
**dadurch gekennzeichnet, daß**
zum Zwecke der Entkeimung der Flüssigkeit (40; 140) eine Elektroporationseinrichtung (54; 154; 254) zum mechanisch-physikalischen Öffnen von zellmembranen in den Kreislauf integriert ist.

2. Oberflächenbehandlungsanlage nach Anspruch 1, **dadurch gekennzeichnet, daß** die Betriebsparameter der Elektroporationseinrichtung (54; 154; 254) während des Betriebs der Oberflächenbehandlungsanlage veränderbar sind.

3. Oberflächenbehandlungsanlage nach Anspruch 1 oder 2, **dadurch gekennzeichnet, daß** in der Elektroporationseinrichtung (254) Koronarentladungen erzeugbar sind.

4. Oberflächenbehandlungsanlage nach einem der vorhergehenden Ansprüche, **dadurch gekennzeichnet, daß** der Kreislauf (44; 144) Teil einer Wiederaufbereitungseinrichtung zum Wiederaufbereiten der Flüssigkeit (40; 140) ist.

5. Oberflächenbehandlungsanlage nach Anspruch 4, **dadurch gekennzeichnet, daß** die Wiederaufbereitungseinrichtung einer Station (30) zur kataphoretischen Tauchlackierung zugeordnet ist.

6. Oberflächenbehandlungsanlage nach Anspruch 4, **dadurch gekennzeichnet, daß** die Wiederaufbereitungseinrichtung einer der Lackierung vorgelagerten Vorbehandlungs-Station (12, 14, 16, 17, 18, 20, 22, 24, 26, 28, 30, 32, 34, 36) zugeordnet ist.

7. Oberflächenbehandlungsanlage nach Anspruch 6, **dadurch gekennzeichnet, daß** die VorbehandlungsStation eine Entfettungsstation (16) ist.

8. Oberflächenbehandlungsanlage nach Anspruch 6, **dadurch gekennzeichnet, daß** die Vorbehandlungsstation eine Phosphatierungs-Station (22) ist.

9. Oberflächenbehandlungsanlage nach Anspruch 6, **dadurch gekennzeichnet, daß** die Vorbehandlungsstation eine Spülstation (36) ist.

10. Oberflächenbehandlungsanlage nach Anspruch 9, **dadurch** gekenntzeichnet, daß in der Spülstation (36) mit entsalztem Wasser gespült wird.

11. Oberflächenbehandlungsanlage nach Anspruch 6, **dadurch gekennzeichnet, daß** die Wiederaufbereitungseinrichtung einer Anlage zur Erzeugung von entsalztem Wasser zugeordnet ist.

12. Oberflächenbehandlungsanlage nach Anspruch 6, **dadurch gekennzeichnet, daß** die Wiederaufbereitungseinrichtung einer Lack-Sprüh-Station zum Aufsprühen von Lacken zugeordnet ist.

13. Oberflächenbehandlungsanlage nach Anspruch 12, **dadurch gekennzeichnet, daß** die in der Wiederaufbereitungseinrichtung Lackauswaschwässer aufbereitet werden.

14. Oberflächenbehandlungsanlage nach einem der vorhergehenden Ansprüche, **dadurch gekennzeichnet, daß** die Flüssigkeit Wasser (40; 140) oder eine wäßrige Lösung ist.

## Claims

1. A surface treatment system, in particular for carrying out the painting, coating, drying and associated preparation of metallic or nonmetallic articles (42), comprising a circuit (44; 144) in which a liquid (40; 140) is circulated,
**characterised in that**,
for the purposes of disinfecting the liquid (40; 140), an electroporation apparatus (54; 154; 254) for mechanically-physically opening cell membranes is incorporated into the circuit.

2. A surface treatment system according to claim 1, **characterised in that** the operating parameters of the electroporation apparatus (54; 154; 254) may be modified.during operation of the surface treatment system.

3. A surface treatment system according to claim 1 or claim 2, **characterised in that** corona discharges can be generated in the electroporation apparatus (254).

4. A surface treatment system according to any one of the preceding claims, **characterised in that** the circuit (44; 144) is part of a reprocessing apparatus for reprocessing the liquid (40; 140).

5. A surface treatment system according to claim 4, **characterised in that** the reprocessing apparatus is assigned to a cataphoretic dip coating station (30).

6. A surface treatment system according to claim 4, **characterised in that** the reprocessing apparatus is assigned to a pretreatment station (12, 14, 16, 17, 18, 20, 22, 24, 26, 28, 30, 32, 34, 36) upstream of painting.

7. A surface treatment system according to claim 6, **characterised in that** the pretreatment station is a degreasing station (16).

8. A surface treatment system according to claim 6, **characterised in that** the pretreatment station is a phosphating station (22).

9. A surface treatment system according to claim 6, **characterised in that** the pretreatment station is a rinsing station (36).

10. A surface treatment system according to claim 9, **characterised in that** rinsing is performed with deionised water in the rinsing station (36).

11. A surface treatment system according to claim 6, **characterised in that** the reprocessing apparatus is assigned to a system for producing deionised water.

12. A surface treatment system according to claim 6, **characterised in that** the reprocessing apparatus is assigned to a paint spraying station for spray application of paints.

13. A surface treatment system according to claim 12, **characterised in that** paint rinsing water is reprocessed in the reprocessing apparatus.

14. A surface treatment system according to any one of the preceding claims, **characterised in that** the liquid is water (40; 140) or an aqueous solution.

## Revendications

1. Installation de traitement de surface, en particulier pour la peinture, le revêtement, le séchage et les préparations associées d'objets métalliques ou non métalliques (42), comprenant un circuit (44 ; 144) dans lequel on fait circuler un liquide (40 ; 140),
**caractérisée en ce que**
dans le but de stériliser le liquide (40 ; 140), un dispositif d'électroporation (54 ; 154; 254) pour l'ouverture mécanico-physique de membranes cellulaires est intégré dans le circuit.

2. Installation de traitement de surface selon la revendication 1, **caractérisée en ce que** les paramètres de fonctionnement du dispositif d'électroporation (54 ; 154 ; 254) sont modifiables pendant le fonctionnement de l'installation de traitement de surface.

3. Installation de traitement de surface selon la revendication 1 ou 2, **caractérisée en ce que** des décharges corona peuvent être produites dans le dispositif d'électroporation (254).

4. Installation de traitement de surface selon l'une des revendications précédentes, **caractérisée en ce que** le circuit (44 ; 144) fait partie d'un dispositif de retraitement servant à retraiter le liquide (40 ; 140).

5. Installation de traitement de surface selon la revendication 4, **caractérisée en ce que** le dispositif de retraitement est associé à une station (30) de peinture cataphorétique par immersion.

6. Installation de traitement de surface selon la revendication 4, **caractérisée en ce que** le dispositif de retraitement est associé à une station de traitement préparatoire (12, 14, 16, 17, 18, 20, 22, 24, 26, 28, 30, 32, 34, 36) en amont de la peinture.

7. Installation de traitement de surface selon la revendication 6, **caractérisée en ce que** la station de traitement préparatoire est une station de dégraissage (16).

8. Installation de traitement de surface selon la revendication 6, **caractérisée en ce que** la station de traitement préparatoire est une station de phosphatation (22).

9. Installation de traitement de surface selon la revendication 6, **caractérisée en ce que** la station de traitement préparatoire est une station de rinçage (36).

10. Installation de traitement de surface selon la revendication 9, **caractérisée en ce que** dans la station de rinçage (36), le rinçage est effectué à l'eau déminéralisée.

11. Installation de traitement de surface selon la revendication 6, **caractérisée en ce que** le dispositif de retraitement est associé à une installation de production d'eau déminéralisée.

12. Installation de traitement de surface selon la revendication 6, **caractérisée en ce que** le dispositif de retraitement est associé à une station de pulvérisation de peinture servant à pulvériser des peintures.

13. Installation de traitement de surface selon la revendication 12, **caractérisée en ce que** des eaux de lavage de peinture sont traitées dans le dispositif de retraitement.

14. Installation de traitement de surface selon l'une des revendications précédentes, **caractérisée en ce que** le liquide est de l'eau (40 ; 140) ou une solution aqueuse.
